(19) Europäisches Patentamt
European Patent Office
Office européen des brevets

(11) Publication number: 0 063 944
B1

(12) # EUROPEAN PATENT SPECIFICATION

(45) Date of publication of patent specification: **10.07.85**

(21) Application number: **82302126.6**

(22) Date of filing: **26.04.82**

(51) Int. Cl.⁴: **C 07 C 69/675,**
C 07 C 101/32, C 07 C 121/34,
C 08 L 75/04, C 07 C 67/31,
C 07 C 102/00, C 07 C 121/00
// C08G18/32

(54) **Polyhydric alcohols.**

(30) Priority: **27.04.81 US 257674**

(43) Date of publication of application:
**03.11.82 Bulletin 82/44**

(45) Publication of the grant of the patent:
**10.07.85 Bulletin 85/28**

(84) Designated Contracting States:
**AT BE CH DE FR GB IT LI LU NL SE**

(56) References cited:
**EP-A-0 017 503**
**FR-A-1 184 093**
**US-A-2 605 270**
**US-A-4 093 637**

(73) Proprietor: **HENKEL CORPORATION**
**7900 West 78th Street**
**Minneapolis Minnesota 55435 (US)**

(72) Inventor: **Rogier, Edgar Robert**
**16400 Hidden Valley Road**
**Minnetonka Minnesota 55343 (US)**

(74) Representative: **Watkins, Arnold Jack et al**
**European Patent Attorney Frank B. Dehn & Co.**
**Imperial House 15-19 Kingsway**
**London WC2B 6UZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 063 944 B1

Courier Press, Leamington Spa, England.

**Description**

The present invention relates to polyhydric alcohols and in particular to polyhydric alcohols having a further polar functional group.

It is now known that certain polyhydric alcohols, particularly those having a *geminal*-bis(hydroxymethyl) functionality are highly useful for curing urethane compositions. Such work is described in the United States patent 4,216,343 of Rogier issued August 5, 1980 which discloses *geminal*-bis(hydroxymethyl) alcohols which also contain a terminal hydroxyl functionality. An example of such a compound is *gem*-bis(hydroxymethyl) octadecanol.

It has been found highly desirable to use such compounds due to the high reactivity of the hydroxyl groups and the stability of the polyol. Additional work concerning such materials is shown in a series of articles from the Northern Regional Research Laboratory (NRRL) of the United States Department of Agriculture at Peoria, Illinois, U.S.A. Representative of such materials worked on by the NRRL are 9,9(10,10)-bis(hydroxymethyl) octadecanoate esters and the corresponding acid. Such work is described by Miller and Pryde [J. Amer. Oil Chemists Soc. *54*, 822A—885A (1977); *ibid*, *55*, 469—470 (1978); U.S. Patent 4,093,637]. US Patent 4093637 in particular discloses the preparation of $C_{1-8}$ alkyl esters, such as the methyl, ethyl and butyl esters, of 9,9(10,10)-bis(hydroxymethyl) octadecanoic acid and describes these esters as being useful as intermediates in the preparation of primary plasticizers for polyvinylchloride. Further disclosure of hydroformylation technology is made in an article entitled *"Obtention de derives biofunctionnels"* by R. Lai in Rev. Fr. Corps Gras. 17:455 (1970) b.

Such compounds, however, have carboxyl groups or hydrolyzable ester groups and are thus not fully usable for wetting pigments in polyurethane coatings. Furthermore, compounds such as those of the present invention are miscible with polyisocyanates used to formulate coating systems. It is, of course, necessary to have sufficient miscibility to allow the formulations of the polyhydroxy compounds of the present invention into polyurethanes. Of course, the compounds of the present invention may be utilized in any area where both a polyhydroxyl functionality and a polar group are required.

Throughout the specification and claims, percentages and ratios are by weight, pressure is gauge, and temperatures are in degrees Celsius unless otherwise indicated.

According to one aspect of the present invention we provide polyhydroxymethyl compounds of formula I

$$CH_3(CH_2)_m[C(CH_2OH)X]_n(CH_2)_p[C(CH_2OH)X]_q(CH_2)_r[C(CH_2OH)X]_s(CH_2)_tCH_2M \qquad (I)$$

(wherein X represents a hydrogen atom or a hydroxymethyl group; M represents a cyano, ester or N,N-disubstituted amide group, the $CH_2$—M bond being a carbon-carbon bond; m, n, p, q, r, s and t are non-negative integers the sum of which is from 11 to 19; and n, q and s each has the value 0 or 1, the sum of n, q and s being 1, 2 or 3; with the provisos that where the sum of n, q and s is 1, X is a hydroxymethyl group, where the sum of n, q and s is 2 or 3, each group represented by X is the same and that where the sum of n, q and s is 1 and the sum of m, p, r and t is 14, M represents a cyano or N,N-disubstituted amide group) and mixtures thereof.

In the compounds of the invention, the polar group M preferably represents a cyano group, an ester group of formula —COOR or an amide group of formula —$CONR_2$ (in which R may be the same or different). The substituent R is an organic radical, preferably a $C_{1-4}$ straight chained or branched alkyl group, optionally substituted by a hydroxyl group, or a phenyl group. Where M represents the amide group —$CONR_2$, the moiety $NR_2$ together may further represent a piperidino or morpholino group.

The sum of m, n, p, q, r, s and t preferably is from 13 to 17. The integers m and t are each preferably at least 3, especially preferably 4, 5, 6 or greater. Where q is 1, it is preferred that n and s are each 0. The requirement for the compound of formula I to contain at least two hydroxymethyl groups (the proviso that where the sum of n, q and s is 1, X represents a hydroxymethyl group) will be satisfied where the molecule contains a gem bis hydroxymethyl group and/or where hydroxymethyl groups are located at different carbon atoms on the carbon chain backbone of the molecule of formula I.

The present invention relates to polyhydroxyl compounds which contain a polar functional group which may be introduced into the molecule by one of several methods. The hydroxyl functionality may be introduced into the molecule by utilizing an unsaturated material which is hydroformylated e.g. by the use of synthesis gas (a carbon monoxide/hydrogen mixture) to introduce a formyl group at the site of the unsaturation. Catalysts may be selected such that a polyunsaturated material is either polyhydroformylated or is monohydroformylated. Rhodium catalysts give products which introduce the formyl group at substantially every location of unsaturation in the molecule. On the other hand, cobalt catalysts introduce a formyl group only at one site of unsaturation in conjugated polyunsaturated materials.

The formyl groups can then be reacted via a Tollens' reaction utilizing a strong base and two moles of formaldehyde per mole of the original formyl group, to convert the formyl group directly to the *geminal*-bis(hydroxymethyl) structures.

However, if it is desired, the formyl group may be converted with selective catalysts such as sodium borohydride to convert the formyl group to a hydroxymethyl group. It is noted, however, that the present

invention requires that if such a reaction is conducted the intermediate aldehyde must contain more than one formyl group to ensure that a polyhydroxymethyl compound will be obtained.

According to a further aspect of the present invention we provide a process for the preparation of polyhydroxymethyl compounds of formula I which process comprises (a) hydroformylating an olefinic compound of formula II

$$CH_3(CH_2)_a(CH{=}CH)_b(CH_2)_c(CH{=}CH)_d(CH_2)_e(CH{=}CH)_f(CH_2)_gCH_2M \qquad (II)$$

(wherein M is as hereinbefore defined; a, b, c, d, e, f and g are non-negative integers, the sum of a, c, e, g, 2b, 2d and 2f being from 11 to 19; and b, d and f each has the value 0 or 1, the sum of b, d and f being 1, 2 or 3) and (b) converting the or each

$$>CH{-}CHO$$

group in the product thereby obtained (i) to a

$$>C(CH_2OH)_2$$

group by reacting with formaldehyde in the presence of a strong base, (ii) to a

$$>CH{-}CH_2OH$$

group by catalytic reduction, or (iii) to a

$$>C(CHO)(CH_2OH)$$

group by reacting with formaldehyde in the presence of a weak base and subsequently to a

$$>C(CH_2OH)_2$$

group be reduction, subject to the proviso that where the product obtained by step (a) contains a single formyl group, the conversion of step (b) is effected by process steps (i) or (iii).

The starting materials of formula II which may be converted into the desired products of this invention include oleonitrile, a commercially available compound. The reactions are shown in the following scheme:

$$CH_3(CH_2)_7CH{=}CH(CH_2)_7CN \xrightarrow[Rh]{CO-H_2} CH_3(CH_2)_{8(7)}CH(CHO)(CH_2)_{7(8)}CN$$

Cat. 9(10)-formyloctadecanonitrile (I)

$$I \xrightarrow[NaOH]{2CH_2O} CH_3(CH_2)_{8(7)}C(CH_2OH)_2(CH_2)_{7(8)}CN$$

9,9(10,10)-bis(hydroxymethyl)octadecanonitrile

In a similar fashion, linoleonitrile can be converted into a tetrahydroxynitrile or a dihydroxy nitrile as shown in the following scheme:

$$CH_3(CH_2)_4CH{=}CH\ CH_2CH{=}CH(CH_2)_7CN$$

linoleonitrile

Rh cat ↓ Co—$H_2$

$$CH_3(CH_2)_xCH(CHO)(CH_2)_yCH(CHO)(CH_2)_zCN$$

where x+y+z=14

4 $CH_2O$ + 2NaOH

$$CH_3(CH_2)_xC(CH_2OH)_2(CH_2)_yC(CH_2OH)_2(CH_2)_zCN$$

$NaBH_4$

$$CH_3(CH_2)_xCH(CH_2OH)(CH_2)_yCH(CH_2OH)(CH_2)_zCN$$

Likewise, linolenonitrile can be converted into the triformyl derivative by hydroformylation using a rhodium catalyst and synthesis gas. A tri [*gem*-bis(hydroxymethyl)] octadecanonitrile can be formed by reacting triformyl nitrile with excess formaldehyde and caustic.

In this invention, unsaturated fatty compounds having 13—23 carbon atoms (excluding those in the polar substituent M) and having the above terminal polar function can be used for the preparation of the polyhydric alcohols of formula I.

Similarly, 9,9(10,10)-bis(hydroxymethyl)-N,N-disubstituted octadecanamide can be obtained from oleic acid as a starting material as illustrated in the following scheme:

$$CH_3(CH_2)_7CH{=}CH(CH_2)_7COOH$$

$HNR_2$

$$CH_3(CH_2)CH{=}CH(CH_2)_7CONR_2{+}H_2O$$

$H_2$ Rh catalyst

CO

$$CH_3(CH_2)_{8(7)}CH(CH_2)_{7(8)}CONR_2$$

CHO

2 $CH_2O$

Na OH

$$CH_3(CH_2)_{8(7)}C(CH_2OH)_2(CH_2)_{7(8)}CONR_2$$

(A 9,9(10,10)-bis(hydroxymethyl)-N,N-disubstituted octadecanamide).

The value of R in the disubstituted amide above may be for example dimethyl, diethyl, diphenyl, dibutyl, piperidene or morpholine. Again, while oleic acid is used as an example of the starting material, linoleic and linolenic acids could alternatively be used.

It has been found that one particular ester of oleic acid may be converted very usefully through the use of isobutylene to give the tertiary butyl ester denominated as t-butyl 9,9(10,10)-bis(hydroxymethyl)octadecanoate. The t-butyl esters of linolenic and linoleic acids may also be employed analogously.

Another class of compounds are noted as starting materials for reacting with synthesis gas to obtain compounds having formyl groups in more than one position in the molecule. This class includes linoleyl or linolenyl acids and alcohols as such starting materials are required to have more than one site of unsaturation. The two or three formyl groups, which result from hydroformylation of a starting material containing two or three carbon-carbon double bonds, may be reduced with sodium borohydride to obtain the di- or tri hydroxymethyl product, e.g. using a linolenyl starting material the product will be, for instance, a 9(10), 12(13) di-hydroxymethyloctadecanonitrile, amide or ester, (such as a tertiary butyl ester).

Suggested values for R are those having from 1 to 4 carbon atoms such as methyl, ethyl, propyl, butyl, sec-butyl, hydroxypropyl, hydroxyethyl or mixtures thereof, and, for the moiety $NR_2$, morpholino and piperidino groups.

To conduct the synthesis gas reaction, hydrogen and carbon monoxide are added in a closed vessel at temperatures of from 100 to 150 degrees C. The hydrogen and carbon monoxide may be maintained

conveniently at molar ratios of from 1.5:0.5 to about 0.5:1.5. It is noted that the ratio is not critical so long as the pressure is maintained in the reaction vessel by the component gases and the amount of hydrogen is not so great as to substantially reduce the unsaturated starting materials.

In practice, hydroformylation with the synthesis gas may be conducted at from about 90 degrees C to about 170 degrees C, preferably from 110 degrees C to about 130 degrees C. Above the higher temperatures described above, increased amounts of unwanted by-products are formed in the reaction mixture. The pressure conditions suitably are maintained in this sealed system at from about 20 to about 500 atmospheres (20.3 to 506.6 bar), preferably from about 30 to about 100 atmospheres absolute (30.4 to 101.3 bar) during the hydroformylation.

Conventional cobalt catalysts may be employed to obtain a single formyl group, but are not particularly suitable for polyunsaturated compounds due to reduction of the unsaturation. Conveniently, however, the practical catalyst to use during the synthesis gas reaction is a rhodium-based catalyst. The rhodium may be in any convenient form such as rhodium metal, rhodium oxide, and various other rhodium salts such as rhodium chloride, rhodium dicarbonyl chloride dimer, rhodium nitrate, rhodium trichloride and other similar materials. The rhodium catalyst is also best utilized with a ligand such as trisubstituted phosphine or trisubstituted phosphite. The term trisubstituted includes both alkyl and aryl compounds and the substituted compounds of alkyl and aryl compounds.

A particularly valuable ligand for the rhodium carbonyl hydride is triphenylphosphite or triphenylphosphine in that both compounds are particularly useful in minimizing migration of the double bond thereby avoiding a large number of isomers with respect to the formyl group. In general, triarylphosphines or triarylphosphites may be used for this purpose in the formation of the rhodium carbonylhydride ligand. In addition, the foregoing materials are extremely valuable in minimizing the undesired saturation of the double bond or the reduction of the formyl group.

As was previously noted, materials such as sodium borohydride may be utilized to reduce the formyl group to a hydroxymethyl group thereby obtaining desired compounds of the invention.

However, in most cases it is desirable to convert the formyl group completely to the *gem*-bis(hydroxymethyl) structure via a Tollens' reaction. Such may be achieved by utilizing a strong base as a reactant. Should it be desired for some reason, weak base and one mole formaldehyde may be used to obtain the hydroxymethyl formyl compound which may be utilized for various purposes including subsequent reduction of the formyl group to give the *gem*-bis(hydroxymethyl) variety of the claimed compounds.

In either case, formaldehyde (which is relatively inexpensive) may be used in for example up to 1.5, preferably up to 1.2 times the amount actually required to obtain the hydroxymethyl formyl or the *gem*-bis(hydroxymethyl) compounds. A convenient manner of adding the formaldehyde and conducting the Tollens' reaction is by using a methanol solution of formaldehyde.

As a strong base sodium hydroxide conveniently may be employed, although potassium or calcium hydroxide may alternatively be employed. The Tollens' reaction is generally conducted at a temperature of from about 0 degrees to about 100 degrees C, preferably from about 20 degrees C to about 70 degrees C.

The compounds of the present invention are particularly suitable for use in reaction with polyisocyanates to form urethanes. A substantial advantage in the compounds of the present invention is that the polar group of the molecule enhances miscibility of the respective urethane forming components. Another significant advantage is the wetting ability of the polar groups which allows for the introduction of pigments such as organic or inorganic pigments, e.g. titanium dioxide, chrome yellow, calcium or barium Lithol, phthocyanines and oxide pigments thereby providing urethane coatings with a high pigment carrying capability.

According to a further aspect of the present invention we therefore provide coating and moulding compositions comprising a compound of formula I or the reaction product thereof with a polyisocyanate.

The following Examples are provided to illustrate the present invention without serving to restrict the scope of protection sought therefor:—

Preparative Example A

Preparation of formyloctadecanonitrile (FON)

Into a 1 litre 316 SS autoclave equipped with stirrer and heat exchange coil is place 617 grams of oleonitrile, 3.0 grams of 5 percent rhodium on aluminium (Englehardt Industries) and 3.2 grams of triphenylphosphite. The autoclave is flushed with nitrogen then pressurized with carbon monoxidehydrogen (1:1) to 1080 psig (7.446 MPa gauge). The temperature is increased to 127—133 degrees C and maintained for 3.3 hours with a CO—$H_2$ pressure of 970—1080 psig (6.688 to 7.446 MPa gauge). At this point a GC analysis indicated complete reaction of oleonitrile with the formation of FON. The reaction was cooled, the pressure vented and the product filtered. The yield of FON is 681 g. Rhodium is removed from the product by vacuum distillation.

Example I

Preparation of gem-bis(hydroxymethyl)octadecanonitrile (BHMON)

Into a 3 litre glass reaction flask is placed 762 grams (2.60 moles) of formyloctadecanonitrile, 321 grams (5.84 moles) of a 54.6 percent solution of formaldehyde in methanol (Methyl Formcel, a product of Celanese

Chemical Company), and 6.6 millilitres of 40 percent solution of sodium hydroxide in water. The temperature of the system is maintained at 40 degrees C for 36 minutes then increased to 50 degrees C.

After 25 minutes, a 40 percent solution of sodium hydroxide in water is added at a rate of 5.3 millilitres/minute until, including the 6.6 millilitres initially added, a total of 297 grams has been added. Temperature is maintained at 45—50 degrees C throughout the addition and then held at 50 degrees C for an additional 2 hours. The reaction mixture was cooled to 16 degrees C and 27.9 grams of a 12 percent solution of sodium borohydride in 43 percent sodium hydroxide is added.

The reaction temperature is maintained between 16—27 degrees C for 20 minutes.

The reaction mixture is stripped under vacuum at 49 degrees C to remove methanol and water. The residual product is washed at 54 degrees C with 500 millilitres of water plus 100 millilitres of saturated sodium sulfate solution. The upper organic phase is separated and washed successively with 500 millilitres of 0.1 N sodium hydroxide solution and 4× with 500 millilitres portions of water. The product is finally dried in partial vacuum (1 mm Hg (1.33 mbar)) at 70 degrees C. The yield of crude BHMON was 832 grams.

Preparative Example B

Preparation of 9(10),12(13)-diformyloctadecanonitrile

Hydroformylation of 9,12-linoleonitrile was carried out similar to conditions described in Preparative Example A, except that the CO—$H_2$ pressure is maintained at 2000 psig (13.79 MPa gauge).

Example II

Preparation of 9(10),12(13)-bis(hydroxymethyl)octadecanonitrile

Into a solution of 8.86 grams of 9(10),12(13)-diformyloctadecanonitrile (containing about 47 percent 9(10)-formyloctadecanonitrile) in 8.3 grams of isopropyl alcohol at 25 degrees C is added 0.77 grams of sodium borohydride over a period of 5 minutes. After 4 hours at 28 degrees C, additional sodium borohydride (9.24 grams) is added. After 44 minutes the reaction is worked up by addition of 5 millilitres acetone, distillation of the volatiles in vacuo. The residue is dissolved in a mixture of ether-water and the ether layer is washed with water. Evaporation of the ether yielded 7.60 grams of 9(10),12(13)-bis(hydroxymethyl) octadecanonitrile containing some 9(10)-hydroxymethyloctadecanonitrile. Identification of the products was made by I.R., NMR and GC-MS.

Example III

Preparation of 9(10),12(13)-bis(hydroxymethyl)octadecanonitrile

Into a solution of 8.86 grams of 9(10),12(13)-diformyloctadecanonitrile (containing about 47 percent 9(10)-formyloctadecanonitrile) in 8.3 grams of isopropyl alcohol at 25 degrees C is added 0.77 grams of sodium borohydride over a period of 5 minutes. After 4 hours at 28 degrees C, additional sodium borohydride (0.24) grams is added. After 44 minutes the reaction is worked up by addition of 5 millilitres acetone and distillation of the volatiles in vacuo. The residue is dissolved in a mixture of ether-water and the ether layer is washed with water. Evaporation of the ether yielded 7.60 grams of 9(10),12(13)-bis(hydroxymethyl)octadecanonitrile. Identification of the products is made by I.R., NMR and GC-MS.

Example IV

Thermoplastic and thermosetting polymers are prepared as shown in the Table.

TABLE I

| NCO Terminated Prepolymer 1 equivalent | Curative 0.95 equivalent | Split tear in psi |
|---|---|---|
| A. BHON/PM 1000 4:1 MDI | BHON/80 1:2 | 1344 (9.27) |
| B. BHON/PM 1000 4:1 MDI | 1,4 BD/BHON/C-20 triol 8:2:1 | 1288 (8.88) |

* Figures in brackets are in MPa.

These products show exceptional split tear strength.

BHON is used to indicate 9(10) gem-bis (hydroxymethyl)octadecanonitrile. MDI is methylene diisocyanate and PM 1000 is PolyMeg 1000 a polyoxytetramethylene glycol. The ratios are in equivalents. BD indicates 1,4 butane diol and C-20 triol is 9(10)gem-bis(hydroxymethyl)octadecanol. A is a thermoplastic elastomer and B is thermosetting elastomer.

**Claims for the Contracting States: BE FR DE IT LU NL SE CH LI GB**

1. Polyhydroxymethyl compounds of formula I

$$CH_3(CH_2)_m[C(CH_2OH)X]_n(CH_2)_p[C(CH_2OH)X]_q(CH_2)_r[C(CH_2OH)X]_s(CH_2)_tCH_2M \qquad (I)$$

(wherein X represents a hydrogen atom or a hydroxymethyl group; M represents a cyano, ester or N,N-disubstituted amide group, the $CH_2$—M bond being a carbon-carbon bond; m, n, p, q, r, s and t are non-negative integers the sum of which is from 11 to 19; and n, q and s each has the value 0 or 1, the sum of n, q and s being 1, 2 or 3; with the provisos that where the sum of n, q and s is 1, X represents a hydroxymethyl group, where the sum of n, q and s is 2 or 3, each group represented by X is the same and that where the sum of n, q, and s is 1 and the sum of m, p, r and t is 14, M represents a cyano or N,N-disubstituted amide group) and mixtures thereof.

2. Compounds of formula I as claimed in claim 1 wherein M is a cyano group.

3. Compounds of formula I as claimed in claim 1 wherein M is a group —COOR or —$CONR_2$ in which R, which may be the same or different where M represents an amide group, represents a $C_{1-4}$ straight chained or branched alkyl group, optionally substituted by a hydroxyl group, or a phenyl group and where M represents an amide group, the $NR_2$ moiety may further represent a piperidino or morpholino group.

4. Compounds of formula I as claimed in any one of claims 1 to 3 for which at least one of the following conditions is satisfied:

(a) the sum of m, n, p, q, r, s and t is from 13 to 17;
(b) q is 1 and n and s are each zero;
(c) m and t are each 3 or greater; and
(d) m and t are each 4 or greater.

5. Compounds of formula I as claimed in claim 1 selected from the following:

(a) 9,9(10,10)-bis(hydroxymethyl) octadecanonitrile;
(b) 9,9(10,10),12,12(13,13)-tetra(hydroxymethyl)octadecanonitrile;
(c) 9,9(10,10),12,12(13,13),15,15(16,16)-hexa(hydroxymethyl)octadecanonitrile;
(d) 9,(10),12(13)-di(hydroxymethyl)octadecanonitrile; and
(e) 9(10),12(13),15(16)-tri(hydroxymethyl)octadecanonitrile.

6. A process for the preparation of polyhydroxymethyl compounds of formula I (as defined in claim 1) which process comprises (a) hydroformylating an olefinic compound of formula II.

$$CH_3(CH_2)_a(CH{=}CH)_b(CH_2)_c(CH{=}CH)_d(CH_2)_e(CH{=}CH)_f(CH_2)_gCH_2M \qquad (II)$$

(wherein M is as defined in claim 1; a, b, c, d, e, f and g are non-negative integers, the sum of a, c, e, g, 2b, 2d and 2f being from 11 to 19; and b, d and f each has the value 0 or 1, the sum of b, d and f being 1, 2 or 3) and (b) converting the or each

$$>CH{-}CHO$$

group in the product thereby obtained (i) to a

$$>C{-}(CH_2OH)_2$$

group by reacting with formaldehyde in the presence of a strong base, (ii) to a

$$>CH\,.\,CH_2OH$$

group by catalytic reduction, or (iii) to a

$$>C(CHO)(CH_2OH)$$

group by reacting with formaldehyde in the presence of a weak base and subsequently to a

$$>C(CH_2OH)_2$$

group by reduction, subject to the proviso that where the product obtained by the step (a) contains a single formyl group, the conversion of step (b) is effected by process steps (i) or (iii).

7. A process as claimed in claim 6 wherein the hydroformylation of step (a) is performed in the presence of a rhodium based catalyst.

8. A process as claimed in claim 6 or claim 7 wherein the starting compound of formula II is an oleyl, linoleyl or linolenyl compound.

9. Coating and moulding compositions comprising a compound of formula I as claimed in claim 1 or the reaction product thereof with a polyisocyanate.

**Claims for the Contracting State: AT**

1. A process for the preparation of polyhydroxymethyl compounds of formula I

$$CH_3(CH_2)_m[C(CH_2OH)X]_n(CH_2)_p[C(CH_2OH)X]_q(CH_2)_r[C(CH_2OH)X]_s(CH_2)_tCH_2M \qquad (I)$$

(wherein X represents a hydrogen atom or a hydroxymethyl group; M represents a cyano, ester or N,N-disubstituted amide group, the $CH_2$—M bond being a carbon-carbon bond; m, n, p, q, r, s and t are non-negative integers the sum of which is from 11 to 19; and n, q and s each has the value 0 or 1, the sum of n, q and s being 1, 2 or 3; with the provisos that where the sum of n, q and s is 1, X represents a hydroxymethyl group, where the sum of n, q and s is 2 or 3, each group represented by X is the same and that where the sum of n, q, and s is 1 and the sum of m, p, r and t is 14, M represents a cyano or N,N-disubstituted amide group) and mixtures thereof, which process comprises (a) hydroformylating an olefinic compound of formula II

$$CH_3(CH_2)_a(CH{=}CH)_b(CH_2)_c(CH{=}CH)_d(CH_2)_e(CH{=}CH)_f(CH_2)_gCH_2M \qquad (II)$$

(wherein M is as defined above; a, b, c, d, e, f and g are non-negative integers, the sum of a, c, e, g, 2b, 2d and 2f being from 11 to 19; and b, d and f each has the value 0 or 1, the sum of b, d and f being 1, 2 or 3) and (b) converting the or each

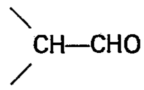

group in the product thereby obtained (i) to a

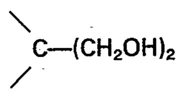

group by reacting with formaldehyde in the presence of a strong base, (ii) to a

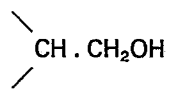

group by catalytic reduction, or (iii) to a

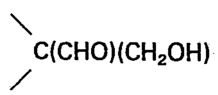

group by reacting with formaldehyde in the presence of a weak base and subsequently to a

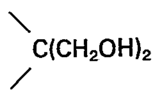

group by reduction, subject to the proviso that where the product obtained by the step (a) contains a single formyl group, the conversion of step (b) is effected by process steps (i) or (iii).

2. A process as claimed in claim 1 wherein the hydroformylation of step (a) is performed in the presence of a rhodium catalyst.

3. A process as claimed in claim 1 or claim 2 wherein the starting compounds of formula II is an oleyl, linoleyl or linolenyl compound.

4. A process as claimed in either of claims 1 and 2 wherein the starting compound of formula II is a compound for which at least one of the following conditions is satisfied:—

(a) M represents a cyano group or a group —COOR or $CONR_2$ in which R, which may be the same or different where M represents an amide group, represents a $C_{1-4}$ straight chain or branched alkyl group, optionally substituted by a hydroxyl group, or a phenyl group and, where M represents an amide group, the $NR_2$ moiety may further represent a piperidino or morpholino group;

(b) the sum of a, c, e, g, 2b, 2d and 2f is from 13 to 17;

(c) b is 1 and d and f are each zero;

(e) a and g are each 3 or greater; and

(f) a and g are each 4 or greater.

**Patentansprüche für die Vertragsstaaten: BE CH DE FR GB IT LI LU NL SE**

1. Polyhydroxymethyl-Verbindungen der Formel I

$$CH_3(CH_2)_m[C(CH_2OH)X]_n(CH_2)_p[C(CH_2OH)X]_q(CH_2)_r[C(CH_2OH)X]_s(CH_2)_tCH_2M \qquad (I)$$

(in der

X ein Wasserstoff-Atom oder eine Hydroxymethyl-Gruppe bezeichnet,

M eine Cyano-, Ester- oder N,N-disubstituierte Amid-Gruppe bezeichnet, wobei die $CH_2$—M -Bindung eine Kohlenstoff-Kohlenstoff-Bindung ist,

m, n, p, q, r, s und t nicht negative ganze Zahlen sind, deren Summe 11 bis 19 beträgt, und n, q und s jeweils den Wert 0 oder 1 haben, wobei die Summe von n, q und s 1, 2 oder 3 ist, mit der Maßgabe, daß, wenn die Summe von n, q und s 1 ist, X eine Hydroxymethyl-Gruppe bezeichnet, und wenn die Summe von n, q und s 2 oder 3 ist, jede durch X bezeichnete Gruppe die gleiche ist, und daß, wenn die Summe von n, q und s 1 ist und die Summe von m, p, r und t 14 ist, M eine Cayno- oder N,N-disubstituierte Amid-Gruppe bezeichnet) und deren Gemische.

2. Verbindungen nach Anspruch 1, dadurch gekennzeichnet, daß M eine Cyano-Gruppe ist.

3. Verbindungen nach Anspruch 1, dadurch gekennzeichnet, daß M eine Gruppe —COOR oder —$CONR_2$ ist, worin R, die beide gleich oder verschieden sein können, wenn M eine Amid-Gruppe bezeichnet, eine geradkettige oder verzweigte $C_{1-4}$-Alkyl-Gruppe bezeichnet, die gegebenenfalls durch eine Hydroxyl-Gruppe oder eine Phenyl-Gruppe substituiert ist, und, wenn M eine Amid-Gruppe bezeichnet, die Gruppe —$NR_2$ weiterhin eine Piperidino- oder Morpholino-Gruppe darstellen kann.

4. Verbindungen nach irgendeinem der Ansprüche 1 bis 3, dadurch gekennzeichnet, daß für sie wenigstens eine der folgenden Bedingungen erfüllt ist:

(a) die Summe von m, n, p, q, r, s und t beträgt 13 bis 17;

(b) q ist 1 und n und s sind jeweils 0;

(c) m und t sind jeweils 3 oder größer; und

(f) m und t sind jeweils 4 oder größer.

5. Verbindungen der Formel I nach Anspruch 1, ausgewählt aus den folgenden:

(a) 9,9(10,10)-Bis(hydroxymethyl)octadecanonitril;

(b) 9,9(10,10),12,12(13,13)-Tetra(hydroxymethyl)octadecanonitril;

(c) 9,9(10,10),12,12(13,13),15,15(16,16)-Hexa(hydroxymethyl)octadecanonitril;

(d) 9(10),12(13)-Di(hydroxymethyl)octadecanonitril und

(e) 9(10),12(13),15(16)-Tri(hydroxymethyl)octadecanonitril.

6. Verfahren zur Herstellung von Polyhydroxymethyl-Verbindungen der Formel I (wie in Anspruch 1 definiert) durch

(a) Hydroformylieren einer olefinischen Verbindung der Formel II

$$CH_3(CH_2)_a(CH{=}CH)_b(CH_2)_c(CH{=}CH)_d(CH_2)_e(CH{=}CH)_f(CH_2)_gCH_2M \qquad (II)$$

(in der

M die in Anspruch 1 angegebene Bedeutung hat,

a, b, c, d, e, f und g nicht-negative ganze Zahlen sind, wobei die Summe aus a, c, e, g, 2b, 2d und 2f 11 bis 19 beträgt und b, d, f jeweils den Wert 0 oder 1 haben, wobei die Summe von b, d und f jeweils 1, 2 oder 3 ist) und

(b) Überführen der oder jeder

$$>CH{-}CHO$$

-Gruppe in dem dadurch erhaltenen Produkt

(i) in eine

$$>C(CH_2OH)_2\text{-Gruppe}$$

durch Reaktion mit Formaldehyd in Gegenwart einer starken Base,

(ii) in eine

\
$CH—CH_2OH$
/

-Gruppe durch katalytische Reduktion oder

(iii) in eine

\
$C(CHO)(CH_2OH)$
/

-Gruppe durch Reaktion mit Formaldehyd in Gegenwart einer schwachen Base und anschließend in eine

\
$C(CH_2OH)_2$
/

-Gruppe durch Reduktion, mit der Maßgabe, daß

wenn das in Schritt (a) erhaltene Produkt eine einzelne Formyl-Gruppe enthält, die Umwandlung des Schrittes (b) mittels der Verfahrensschritte (i) oder (iii) durchgeführt wird.

7. Verfahren nach Anspruch 6, dadurch gekennzeichnet, daß die Hydroformylierung gemäß Schritt (a) in Gegenwart eines Rhodium-Katalysators durchgeführt wird.

8. Verfahren nach Anspruch 6 oder Anspruch 7, dadurch gekennzeichnet, daß die Ausgangs-Verbindung der Formel (II) eine Oleyl-, Linoleyl- oder Linolenyl-Verbindung ist.

9. Anstrichmittel oder Formmassen, enthaltend eine Verbindung der Formel I nach Anspruch 1 oder deren Reaktionsprodukt mit einem Polyisocyanat.

**Patentansprüche für den Vertragsstaat AT:**

1. Verfahren zur Herstellung von Polyhydroxymethyl-Verbindungen der Formel I

$$CH_3(CH_2)_m[C(CH_2OH)X]_n(CH_2)_p[C(CH_2OH)X]_q(CH_2)_r[C(CH_2OH)X]_s(CH_2)_tCH_2M \qquad (I)$$

(in der

X ein Wasserstoff-Atom oder eine Hydroxymethyl-Gruppe bezeichnet,

M eine Cyano-, Ester- oder N,N-disubstituierte Amid-Gruppe bezeichnet, wobei die $CH_2—M$ -Bindung eine Kohlenstoff-Kohlenstoff-Bindung ist,

m, n, p, q, r, s und t nicht negative ganze Zahlen sind, deren Summe 11 bis 19 beträgt, und n, q und s jeweils den Wert 0 oder 1 haben, wobei die Summe von n, q und s 1, 2 oder 3 ist, mit der Maßgabe, daß, wenn die Summe von n, q und s 1 ist, X eine Hydroxymethyl-Gruppe bezeichnet, und wenn die Summe von n, q und s 2 oder 3 ist, jede durch X bezeichnete Gruppe die gleiche ist, und daß, wenn die Summe von n, q und s 1 ist und die Summe von m, p, r und t 14 ist, M eine Cyano- oder N,N-disubstituierte Amid-Gruppe bezeichnet) und deren Gemischen durch

(a) Hydroformylieren einer olefinischen Verbindung der Formel II

$$CH_3(CH_2)_a(CH{=}CH)_b(CH_2)_c(CH{=}CH)_d(CH_2)_e(CH{=}CH)_f(CH_2)_gCH_2M \qquad (II)$$

(in der

M die im Vorstehenden angegebene Bedeutung hat,

a, b, c, d, e, f und g nicht-negative ganze Zahlen sind, wobei die Summe aus a, c, e, g, 2b, 2d und 2f 11 bis 19 beträgt und b, d, f jeweils den Wert 0 oder 1 haben, wobei die Summe von b, d und f jeweils 1, 2 oder 3 ist) und

(b) Überführen der oder jeder

\
$CH—CHO$
/

-Gruppe in dem dadurch erhaltenen Produkt

(i) in eine

\
$C(CH_2OH)_2$
/

-Gruppe durch Reaktion mit Formaldehyd in Gegenwart einer starken Base,
(ii) in eine

\
$CH—CH_2OH$
/

-Gruppe durch katalytische Reduktion oder
(iii) in eine

\
$C(CHO)(CH_2OH)$
/

-Gruppe durch Reaktion mit Formaldehyd in Gegenwart einer schwachen Base und anschließend in eine

\
$C(CH_2OH)_2$
/

-Gruppe durch Reduktion, mit der Maßgabe, daß

wenn das in Schritt (a) erhaltene Produkt eine einzelne Formyl-Gruppe enthält, die Umwandlung des Schrittes (b) mittels der Verfahrensschritte (i) oder (iii) durchgeführt wird.

2. Verfahren nach Anspruch 1, dadurch gekennzeichnet, daß die Hydroformylierung gemäß Schritt (a) in Gegenwart eines Rhodium-Katalysators durchgeführt wird.

3. Verfahren nach Anspruch 1 oder Anspruch 2, dadurch gekennzeichnet, daß die Ausgangs-Verbindung der Formel (II) eine Oleyl-, Linoleyl- oder Linolenyl-Verbindung ist.

4. Verfahren nach Anspruch 1 und Anspruch 2, dadurch gekennzeichnet, daß die Ausgangs-Verbindung der Formel (II) eine Verbindung ist, für die wenigstens eine der folgenden Bedingungen erfüllt ist:

(a) M bezeichnet eine Cyano-Gruppe oder eine Gruppe —COOR oder —$CONR_2$, worin R, die beide gleich oder verschieden sein können, wenn M eine Amid-Gruppe bezeichnet, eine geradkettige oder verzweigte $C_{1-4}$-Alkyl-Gruppe bezeichnet, die gegebenenfalls durch eine Hydroxyl-Gruppe oder eine Phenyl-Gruppe substituiert ist, und, wenn M eine Amid-Gruppe bezeichnet, die Gruppe —$NR_2$ weiterhin eine Piperidino- oder Morpholino-Gruppe darstellen kann;
(b) die Summe von a, c, e, g, 2b, 2d und 2f beträgt 13 bis 17;
(c) bis ist 1 und d und f sind jeweils 0;
(e) a und g sind jeweils 3 oder größer; und
(f) a und g sind jeweils 4 oder größer.

**Revendications pour les Etats Contractants BE FR DE IT LU NL SE CH LI GB**

1. Composés polyhydroxyméthyles répondant à la formule I

$$CH_3(CH_2)_m[C(CH_2OH)X]_n(CH_2)_p[C(CH_2OH)X]_q(CH_2)_r[C(CH_2OH)X]_s(CH_2)_tCH_2M \qquad (I)$$

(dans laquelle X représente un atome d'hydrogène ou un groupe hydroxyméthyle; M représente un groupe cyano, ester ou amide N,N-disubstitué, la liaison $CH_2$—M étant une liaison carbone-carbone; m, n, p, q, r, s et t sont des nombres entiers non négatifs dont la somme est comprise entre 11 et 19 et n, q et s représentent chacun 0 ou 1, la somme de n, q et s étant 1, 2 ou 3; sous réserve que, dans le cas où la somme de n, q et s est 1, X représente un groupe hydroxyméthyle, où la somme de n, q et s est 2 ou 3, chaque groupe représenté par X est le même et que, dans le cas où la somme de n, q et s est 1 et la somme de m, p, r et t est 14, M représente un groupe cyano ou amide N,N-disubstitué) et leurs mélanges.

2. Composés de formule I selon la revendication 1, caractérisés en ce que M est un groupe cyano.

3. Composés de formule I selon la revendication 1, caractérisés en ce que M est un groupe —COOR ou —$CONR_2$ dans lequel R, qui peut être identique ou différent lorsque M représente un groupe amide, représente un groupe alkyle en $C_{1-4}$ à chaîne droite ou ramifiée, éventuellement substitué par un groupe hydroxyle, ou un groupe phényle et, lorsque M représente un groupe amide, le groupe $NR_2$ peut en outre représenter un groupe pipéridino ou morpholino.

4. Composés de formule I tels que revendiqués dans l'une quelconque des revendications 1 à 3 pour lesquels l'une au moins des conditions suivantes est remplie:
a) la somme de m, n, p, q, r, s et t est comprise entre 13 et 17;
b) q est 1 et n et s sont chacun zéro;
c) m et t sont chacun 3 ou plus; et
d) m et t sont chacun 4 ou plus.

5. Composés de formule I selon la revendication 1, choisis parmi les suivants:
a) 9,9(10,10)-bis(hydroxyméthyl) octadécanonitrile;
b) 9,9(10,10),12,12(13,13)-tétra(hydroxyméthyl)octadécanonitrile;
c) 9,9(10,10),12,12(13,13),15,15(16,16)-hexa(hydroxyméthyl)octadécanonitrile;
d) 9(10),12(13)-di(hydroxyméthyl)octadécanonitrile; et
e) 9(10),12(13),15(16)-tri(hydroxyméthyl)octadécanonitrile.

6. Procédé de préparation de composés polyhydroxyméthyles de formule I (tels que définis dans la revendication 1) consistant a) à hydroformyler un composé oléfinique de formule II

$$CH_3(CH_2)_a(CH{=}CH)_b(CH_2)_c(CH{=}CH)_d(CH_2)_e(CH{=}CH)_f(CH_2)_gCH_2M \quad (II)$$

(dans laquelle M est tel que défini dans la revendication 1; a, b, c, d, e, f et g sont des nombres entiers non négatifs, la somme de a, c, e, g, 2b, 2d et 2f étant comprise entre 11 et 19; et b, d et f étant chacun 0 ou 1, la somme de b, d et f étant 1, 2 ou 3) et b) à transformer le ou chaque groupe

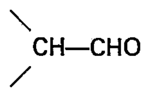

dans le produit ainsi obtenu, (i) en un groupe

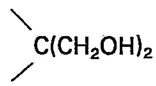

par réaction avec du formaldéhyde en présence d'une base forte, (ii) en un groupe

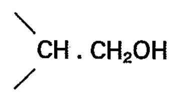

par réduction catalytique ou (iii) en un groupe

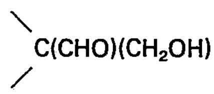

par réaction avec du formaldéhyde en présence d'une base faible et ensuite en un groupe

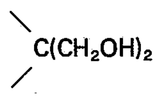

par réduction, à condition que, lorsque le produit obtenu dans l'étape a) renferme un seul groupe formyle, la transformation de l'étape b) s'effectue par la méthode (i) ou (iii).

7. Procédé selon la revendication 6, caractérisé en ce que l'hydroformylation de l'étape (a) est effectuée en présence d'un catalyseur à base de rhodium.

8. Procédé selon la revendication 6 ou 7, caractérisé en ce que le composé de départ de formule II est un composé oléyle, linoléyle ou linolényle.

9. Compositions de revêtement et de moulage contenant un composé de formule I selon la revendication 1 ou son produit de réaction avec un polyisocyanate.

**Revendications pour l'Etat Contractant AT**

1. Procédé de préparation de composés polyhydroxyméthles répondant à la formule I

$$CH_3(CH_2)_m[C(CH_2OH)X]_n(CH_2)_p[C(CH_2OH)X]_q(CH_2)_r[C(CH_2OH)X]_s(CH_2)_tCH_2M \quad (I)$$

(dans laquelle X représente un atome d'hydrogène ou un groupe hydroxyméthyle; M représente un groupe cyano, ester ou amide N,N-disubstitué, la liaison $CH_2$—M étant une liaison carbone-carbone; m, n, p, q, r, s et t sont des nombres entiers non négatifs dont la somme est comprise entre 11 et 19; et n, q et s sont chacun 0 ou 1, la somme de n, q et s étant 1, 2 ou 3; sous réserve que, dans le cas où la somme de n, q et s est 1, X représente un groupe hydroxyméthyle, où la somme de n, q et s est 2 ou 3, chaque groupe représenté par X est le même et que, dans le cas où la somme de n, q et s est 1 et la somme de m, p, r et t est

14, M représente un groupe cyano ou amide N,N-disubstitué) et leurs mélanges, ledit procédé consistant a) à hydroformyler un composé oléfinique de formule II

$$CH_3(CH_2)_a(CH{=}CH)_b(CH_2)_c(CH{=}CH)_d(CH_2)_e(CH{=}CH)_f(CH_2)_gCH_2M \qquad (II)$$

(dans laquelle M est tel que défini ci-dessus; a, b, c, d, e, f, et g sont des nombres entiers non négatifs, la somme de a, c, e, g, 2b, 2d et 2f étant comprise entre 11 et 19; et b, d et f sont chacun 0 ou 1, la somme de b, d et f étant 1, 2 ou 3) et b) à transformer le ou chaque groupe

$$>CH—CHO$$

du produit ainsi obtenu, (i) en un groupe

$$>C(CH_2OH)_2$$

par réaction avec du formaldéhyde en présence d'une base forte, (ii) en un groupe

$$>CH\,.\,CH_2OH$$

par réduction catalytique ou (iii) en un groupe

$$>C(CHO)(CH_2OH)$$

par réaction avec du formaldéhyde en présence d'une base faible et ensuite en un groupe

$$>C(CH_2OH)_2$$

par réduction, à condition que, lorsque le produit obtenu dans l'étape a) renferme un seul groupe formyle, la transformation de l'étape b) s'effectue par la méthode (i) ou (iii).

2. Procédé selon la revendication 1, caractérisé en ce que l'hydroformylation de l'étape (a) est effectuée en présence d'un catalyseur à base de rhodium.

3. Procédé selon la revendication 1 ou 2, caractérisé en ce que le composé de départ de formule II est un composé oléyle, linoléyle ou linolényle.

4. Procédé selon l'une des revendications 1 ou 2, caractérisé en ce que le composé de départ de formule II est un composé pour lequel l'une au moins des conditions suivantes est remplie:

a) M représente un groupe cyano ou un groupe —COOR ou $CONR_2$ dans lequel R, qui peut être identique ou différent lorsque M représente un groupe amide, représente un groupe alkyle en $C_1$—$C_4$ à chaîne droite ou ramifiée, éventuellement substitué par un groupe hydroxyle, ou un groupe phényle et lorsque M représente un groupe amide, le groupe $NR_2$ peut en outre représenter un groupe pipéridino ou morpholino;

b) la somme de a, c, e, g, 2b, 2d et 2f est comprise entre 13 et 17;

c) b est 1 et d et f sont chacun zéro;

e) a et g sont chacun 3 ou plus; et

f) a et g sont chacun 4 ou plus.